(19) Europäisches Patentamt
European Patent Office
Office européen des brevets

(11) EP 0 644 179 B1

(12) # FASCICULE DE BREVET EUROPEEN

(45) Date de publication et mention de la délivrance du brevet: **15.01.1997 Bulletin 1997/03**

(21) Numéro de dépôt: **93420375.3**

(22) Date de dépôt: **17.09.1993**

(51) Int. Cl.[6]: **C07C 219/06**, C07C 231/14, B01F 17/18, C11D 1/62, D06M 13/463, C07C 213/06, C07C 69/24, C07C 69/28, C07C 69/30, C07C 69/33

(54) **Base tensioactive comprenant un composé cationique d'ammonium quaternaire et un composé à titre de solvant et composition assouplissante comprenant une telle base**

Basische Verbindung als Tenside die eine quaternär Ammonium Verbindung und Lösungsmittel umfasst, und ihre umfassende weichrachende Zusammensetzung

Detergent base comprising a cationic quaternary ammonium compound and a solvent compound, and softening composition comprising said base

(84) Etats contractants désignés:
**AT BE CH DE DK ES FR GB GR IE IT LI LU MC NL PT SE**

(43) Date de publication de la demande:
**22.03.1995 Bulletin 1995/12**

(73) Titulaire: **STEPAN EUROPE**
**F-38340 Voreppe (FR)**

(72) Inventeurs:
- **Contet, Jean-Pierre**
  **F-38430 Saint Jean de Moirans (FR)**
- **Courdavault Duprat, Stéphane**
  **F-38240 Meylan (FR)**

(74) Mandataire: **Guerre, Dominique et al**
**Cabinet Germain et Maureau,**
**12, rue Boileau,**
**BP 6153**
**69466 Lyon Cedex 06 (FR)**

(56) Documents cités:

| | |
|---|---|
| EP-A- 0 075 168 | EP-A- 0 131 865 |
| EP-A- 0 284 036 | EP-A- 0 550 361 |
| WO-A-91/15455 | WO-A-91/17974 |
| DE-A- 2 641 286 | US-A- 2 589 674 |
| US-A- 2 935 474 | US-A- 3 915 867 |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Rank Xerox (UK) Business Services
2.13.13/3.4

## Description

L'invention relève du domaine des agents de surface à pouvoir d'adsorption élevé sur les fibres notamment fibres textiles, cheveux, et plus particulièrement des agents de surface cationiques de la classe des ammoniums quaternaires.

L'invention s'intéresse plus spécifiquement à l'application de tels agents en tant qu'adoucissant textile, mais la portée de l'invention n'est nullement limitée à une telle application ; les produits décrits possédant un effet conditionnant vis-à-vis de la plupart des fibres naturelles telles que coton, laine, cheveux, ils sont utilisés dans différents domaines comme agent antistatique, adoucissant et/ou lubrifiant.

Dans la demande de brevet européen, EP-A-0 550 361, déposée au nom de la Demanderesse et dont le contenu est intégré à la présente demande si besoin est, on décrit une base tensioactive comprenant, à titre d'agent de surface, au moins un composé cationique d'ammonium quaternaire répondant à la formule I suivante :

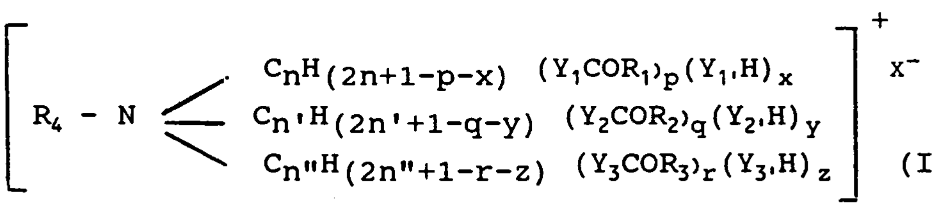
(I)

dans laquelle :

- n, n' n'' sont des nombres entiers non nuls
- p, q, r, x, y, z sont des nombres entiers ou zéro
- la somme de n, n' et n'' est comprise entre 4 et 12
- la somme de p, q, r, x, y et z est comprise entre 1 et 4
- la somme de p, q et r est comprise entre 1,40 et 1,85
- $Y_1$, $Y_{1'}$, $Y_2$, $Y_{2'}$, $Y_3$, $Y_{3'}$, sont des groupes fonctionnels choisis chacun parmi les groupes oxy, N et NH
- $R_1$, $R_2$, $R_3$ sont respectivement une longue chaîne hydrocarbonée alkyle ou alkényle, ayant de 5 à 23 atomes de carbone
- $R_4$ est une courte chaîne alkyle ayant de 1 à 5 atomes de carbone, et
- X est un contre-ion choisi notamment parmi les halogénures et les carbonates et sulfates d'alkyle,

et comprenant à titre de solvant au moins un composé répondant à la formule II suivante:

$$(R_5 - E)_m - C_j\, H_{2(j+1)-m-2d+i}\, O_{k+i} \qquad \text{(II)}$$

dans laquelle :

- $R_5$ est une longue chaîne hydrocarbonée saturée ou insaturée, ayant de 5 à 23 atomes de carbone
- E est une fonction

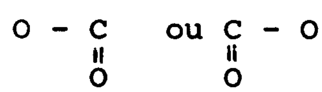

- m est un nombre entier compris entre 1 et 8
- j est un nombre entier compris entre 1 et 36
- k est un nombre entier ou zéro
- i est un nombre entier ou zéro
- d est égal à 0, 1 ou 2.

Selon la demande précitée, le composé cationique d'ammonium quaternaire peut être obtenu par condensation d'une fraction d'amine(s) tertiaire(s) sur une fraction d'acides gras, le rapport molaire de la fraction d'acides gras à celle d'amine(s) tertiaire(s), défini par la somme de p, q et r dans la formule I étant comprise entre 1,40 et 1,85.

En dehors de cet intervalle de valeur, le composé I, en présence des solvants couramment utilisés comme ceux choisis parmi les composés polaires ayant un ou plusieurs groupements hydroxyle et notamment parmi les alcools inférieurs tels que méthanol, éthanol, isopropanol, parmi les glycols tels que éthylène-glycol, propylène-glycol, diéthylène-glycol, butyl-, propyl-, éthyl-, méthyl-éther de diméthylène glycol, de diéthylène glycol, de dipropylène glycol, hexylène-glycol, conduit à des formulations peu efficaces ou à des formulations moins fluides et moins stables.

Selon la présente invention, la Demanderesse s'est aperçu qu'en présence du composé II, à titre de solvant, le composé I conserve de bonnes propriétés conditionnantes ou adoucissantes, même en-dehors dudit intervalle de valeurs compris entre 1,40 et 1,85.

Ainsi le premier objet de l'invention est une base tensioactive qui comprend au moins un composé I répondant à la formule I telle que définie précédemment et au moins un composé II répondant à la formule II telle que définie précédemment, la somme de p, q et r dans la formule I étant comprise entre 1 et 3, à l'exclusion des valeurs comprises entre 1,40 et 1,85.

On a remarqué que pour cette sélection également, de valeurs du rapport de la fraction acides gras/amine, du composé I, le composé II apporte en plus de son pouvoir dissolvant, un effet lubrifiant et un effet conditionnant complémentaires de ceux apportés par le composé I.

Selon l'invention, le composé II préférentiel ne comprend pas de groupements hydroxyle libre, c'est-à-dire i est égal à zéro.

Avantageusement, le composé répondant à la formule II est choisi parmi ceux pour lesquels :

- m est égal à 1, j est compris entre 1 et 18 et de préférence entre 1 et 4, et k, i et d sont égaux à 0, c'est-à-dire notamment parmi les esters méthyliques, éthyliques, propyliques, isopropyliques et butyliques ; pour des valeurs de m et j égales à 1, $R_5$ est une chaîne hydrocarbonée ayant de préférence 7 à 17 atomes de carbone
- m est égal à 2, j est compris entre 2 et 12 et est de préférence égal à 2 ou 3, et k, i et d sont égaux à 0, c'est-à-dire notamment parmi les diesters d'éthylène glycol et de propylène glycol
- m est égal à 2, j est compris entre 4 et 12, k est égal à 1, i et d sont égaux à 0 et $R_5$ est une chaîne hydrocarbonée ayant 7 ou 9 atomes de carbone, tels que l'ester de diéthylèneglycol de l'acide caprique ou caprylique
- m est égal à 3, j est égal à 3 ou 4, k, i et d sont égaux à 0, et $R_5$ est une chaîne hydrocarbonée ayant 7 ou 9 atomes de carbone, tels que le triester de glycérol de l'acide caprique ou caprylique
- m est égal à 3, j est égal à 6 et k, i et d sont égaux à 0, tels que le triester de triméthylol-propane
- m est égal à 4, j est égal à 5, et k, i et d sont égaux à 0, tels que le tétraester de pentaérythritol.

Une base tensioactive de l'invention comprend avantageusement 60 à 99% en poids d'au moins un composé répondant à la formule I et 40 et 1% en poids d'au moins un composé de la formule II.

Un second objet de l'invention est une composition assouplissante qui comprend une base tensioactive de l'invention, c'est-à-dire au moins un composé I et au moins un composé II, tels que définis selon la présente invention.

Les Exemples 1 à 3 suivants sont des exemples de bases tensioactives de l'invention préparées à partir des composés I et II. Ces bases présentent d'excellentes propriétés assouplissantes, et peuvent être, en outre, utilisées à des températures suffisamment faibles en raison de l'effet d'abaissement de la température de trouble desdites bases, par le composé II.

**EXEMPLE 1**

Bases tensioactives de l'invention préparées à partir :

- des composés de formule I dans laquelle, statistiquement :

$$n + n' + n'' = 6,\ x + y + z = 1 \text{ et } p + q + r = 2$$

  préparé par réaction de deux moles d'acide oléique avec une mole de triéthanolamine puis réaction sur le produit intermédiaire obtenu, d'une mole de sulfate de diméthyle, et
- d'un composé de formule II dans laquelle :

  * soit j = 1, m = 1, k = i = d = 0 et $R_5$ a de 15 à 17 atomes de carbone et le composé II est l'ester méthylique d'acide gras de palme (EMAGP) (formulations 3 et 4)
  * soit j = 3, m = 2, k = i = d = 0 et $R_5$ a 7 ou 9 atomes de carbone et le composé II est le di(caprate/caprylate, 65/35, m/m) de propylène glycol (DCCPG) (formulations 7 et 8)
  * soit j = 3, m = 3, k = i = d = 0 et $R_5$ a 7 ou 9 atomes de carbone et le composé II est le tri(caprate/caprylate, 65/35, m/m) de glycérol (TCCG) (formulations 5 et 6).
  * soit j = 3, m = 3, k = i = d = 0 et $R_5$ a de 5 à 17 atomes de carbone et le composé II est l'huile de coprah (HC) (formulations 1 et 2).

Le Tableau 1 présente les formulations 1 à 8 de bases selon l'invention.

TABLEAU 1

| fomulation | 1 | 2 | 3 | 4 | 5 | 6 | 7 | 8 |
|---|---|---|---|---|---|---|---|---|
| Composé I | 90 | 85 | 90 | 85 | 90 | 85 | 90 | 85 |
| EMAGP | 0 | 0 | 10 | 15 | 0 | 0 | 0 | 0 |
| DCCPG | 0 | 0 | 0 | 0 | 0 | 0 | 10 | 15 |
| TCCG | 0 | 0 | 0 | 0 | 10 | 15 | 0 | 0 |
| HC | 10 | 15 | 0 | 0 | 0 | 0 | 0 | 0 |

**EXEMPLE 2**

Bases tensioactives de l'invention préparées à partir :

- des composés de formule I dans laquelle, statistiquement :

$$n + n' + n'' = 6,\ x + y + z = 1 \text{ et } p + q + r = 2$$

préparé par réaction de deux moles d'acide gras de suif partiellement hydrogéné (indice d'iode = 45g $I_2$/100g) avec une mole de triéthanolamine, puis réaction sur le produit intermédiaire obtenu, d'une mole de sulfate de diméthyle, et

- d'un composé de formule II dans laquelle :

  * soit j = 1, m = 1, k = i = d = 0 et $R_5$ a de 5 à 17 atomes de carbone et le composé II est l'ester méthylique des acides gras de coprah (EMC) (formulations 9 et 10)
  * soit j = 3, m = 2, k = i = d = 0 et $R_5$ a 7 ou 9 atomes de carbone et le composé II est le di(caprate/caprylate, 65/35, m/m) de propylène glycol (DCCPG) (formulation 11)
  * soit j = 3, m = 3, k = i = d = 0 et $R_5$ a de 5 à 17 atomes de carbone et le composé II est l'huile de coprah (HC) (formulation 14)
  * soit j = 5, m = 4, k = i = d = 0 et $R_5$ a 7 ou 9 atomes de carbone et le composé II est le tétra(caprate/caprylate, 65/35, m/m) de pentaérythritol (TCCPE) (formulation 12)
  * soit j = 6, m = 3, k = i = d = 0 et $R_5$ a 7 ou 9 atomes de carbone et le composé II est le tri(caprate/caprylate, 65/35, m/m) de triméthylol-propane (TCCT) (formulation 13).

Le Tableau 2 présente les formulations 9 à 14 de bases selon l'invention.

TABLEAU 2

| Formulation | 9 | 10 | 11 | 12 | 13 | 14 |
|---|---|---|---|---|---|---|
| Composé I | 85 | 60 | 80 | 80 | 70 | 70 |
| EMC | 15 | 40 | 0 | 0 | 0 | 0 |
| DCCPG | 0 | 0 | 20 | 0 | 0 | 0 |
| TCCPE | 0 | 0 | 0 | 20 | 0 | 0 |
| TCCT | 0 | 0 | 0 | 0 | 30 | 0 |
| HC | 0 | 0 | 0 | 0 | 0 | 30 |

**EXEMPLE 3**

Bases tensioactives de l'invention préparées à partir :

- d'un composé de formule I dans laquelle :

$$n + n' + n'' = 7,\ x = y = z = 0 \text{ et } p + q + r = 2$$

préparé par réaction de deux moles d'acide gras de suif hydrogéné avec une mole de dihydroxy-1,2- diéthylamino-1-propane, puis réaction sur le produit intermédiaire obtenu, d'une mole de sulfate de diméthyle, et

- d'un composé de formule II dans laquelle :

  * soit j = 1, m = 1, k = i = d= 0 et $R_5$ a 5 ou 17 atomes de carbone et le composé est l'ester méthylique des acides gras de coprah (EMC) (formulations 15, 16 et 17)
  * soit j = 3, m = 2, k = i = d = 0 et $R_5$ a 7 ou 9 atomes de carbone et le composé est le di(caprate/caprylate, 65/35, m/m) de propylène glycol (DCCPG) (formulations 18, 19, 20).

Le Tableau 3 présente les formulations 15 à 20 de bases selon l'invention.

TABLEAU 3

| formulation | 15 | 16 | 17 | 18 | 19 | 20 |
|---|---|---|---|---|---|---|
| Composé I | 90 | 80 | 70 | 90 | 80 | 70 |
| EMC | 10 | 20 | 30 | - | - | - |
| DCCPG | - | - | - | 10 | 20 | 30 |

**Revendications**

1. Base tensioactive comprenant au moins un composé cationique d'ammonium quaternaire répondant à la formule I suivante :

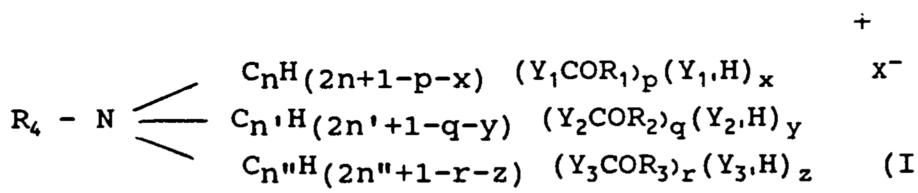

(I)

dans laquelle :

- n, n' n'' sont des nombres entiers non nuls
- p, q, r, x, y, z sont des nombres entiers ou zéro
- la somme de n, n' et n'' est comprise entre 4 et 12
- la somme de p, q, r, x, y et z est comprise entre 1 et 4
- $Y_1$, $Y_{1'}$, $Y_2$, $Y_{2'}$, $Y_3$, $Y_{3'}$, sont des groupes fonctionnels choisis chacun parmi les groupes oxy, N et NH
- $R_1$, $R_2$, $R_3$ sont respectivement une longue chaîne hydrocarbonée alkyle ou alkényle, ayant de 5 à 23 atomes de carbone
- $R_4$ est une courte chaîne alkyle ayant de 1 à 5 atomes de carbone, et
- X est un contre-ion choisi notamment parmi les halogénures et les carbonates et sulfates d'alkyle,

ladite base comprenant à titre de solvant, au moins un composé répondant à la formule II suivante:

$$(R_5 - E)_m - C_j\, H_{2(j+1)-m-2d+i}\, O_{k+i} \qquad (II)$$

dans laquelle :

- $R_5$ est une longue chaîne hydrocarbonée saturée ou insaturée, ayant de 5 à 23 atomes de carbone
- E est une fonction

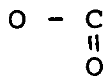 ou 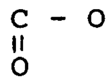

- m est un nombre entier compris entre 1 et 8
- j est un nombre entier compris entre 1 et 36
- k est un nombre entier ou zéro
- i est un nombre entier ou zéro
- d est égal à 0, 1 ou 2,

caractérisée en ce que le composé cationique d'ammonium quaternaire répond à la formule I dans laquelle en outre, la somme de p, q et r est comprise entre 1 et 3, à l'exclusion des valeurs comprises entre 1,40 et 1,85.

**2.** Base selon la revendication 1, caractérisée en ce que i est égal à 0.

**3.** Base selon la revendication 2, caractérisée en ce que si m est égal à 1, j est compris entre 1 et 18 et k et d sont égaux à 0.

**4.** Base selon la revendication 3, caractérisée en ce que j est compris entre 1 et 4.

**5.** Base selon la revendication 4, caractérisée en ce que si j est égal à 1, $R_5$ est une chaîne hydrocarbonée ayant de 7 à 17 atomes de carbone.

**6.** Base selon la revendication 2, caractérisée en ce que si m est égal à 2, k et d sont égaux à 0 et j est compris entre 2 et 12.

**7.** Base selon la revendication 2, caractérisée en ce que si m est égal à 2, k est égal à 1, j est compris entre 4 et 12, d est égal à 0, et $R_5$ est une chaîne hydrocarbonée ayant 7 ou 9 atomes de carbone.

**8.** Base selon la revendication 2, caractérisée en ce que m est égal à 3, j est égal à 3 ou 4, k et d sont égaux à 0 et $R_5$ est une chaîne hydrocarbonée ayant 7 ou 9 atomes de carbone.

**9.** Base selon la revendication 2, caractérisée en ce que si m est égal à 3, j est égal à 6 et k et d sont égaux à 0.

**10.** Base selon la revendication 2, caractérisée en ce que si m est égal à 4, j est égal à 5 et k et d sont égaux à 0.

**11.** Base selon l'une quelconque des revendications 1 à 10, caractérisée en ce qu'elle comprend 60 à 99 % en poids d'au moins un composé de la formule I et 40 à 1 % en poids d'au moins un composé de la formule II.

**12.** Composition assouplissante caractérisée en ce qu'elle comprend une base tensioactive selon l'une quelconque des revendications 1 à 11.

## Claims

**1.** Surfactant base comprising at least one cationic quaternary ammonium compound corresponding to the following formula I:

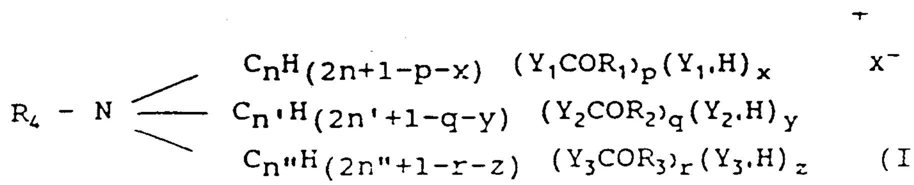

(I)

in which:

- n, n' n" are non-zero integers
- p, q, r, x, y, z are integers or zero
- the sum of n, n' and n" is between 4 and 12
- the sum of p, q, r, x, y and z is between 1 and 4
- $Y_1$, $Y_{1'}$, $Y_2$ $Y_{2'}$, $Y_3$, $Y_{3'}$ are functional groups each chosen from oxy, N and NH groups
- $R_1$, $R_2$, $R_3$ are, respectively, a long alkyl or alkenyl hydrocarbon chain having from 5 to 23 carbon atoms
- $R_4$ is a short alkyl chain having from 1 to 5 carbon atoms, and
- X is a counterion chosen, in particular, from halides and alkyl carbonates and sulphates,

the said base comprising, as a solvent, at least one compound corresponding to the following formula II:

$$(R_5 - E)_m - C_j H_{2(j+1)-m-2d+i} O_{k+1} \qquad (II)$$

in which:

- $R_5$ is a long, saturated or unsaturated hydrocarbon chain having from 5 to 23 carbon atoms
- E is an

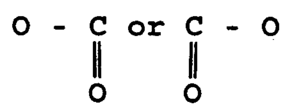

- m is an integer between 1 and 8
- j is an integer between 1 and 36
- k is an integer or zero
- i is an integer or zero
- d is equal to 0, 1 or 2,

characterized in that the cationic quaternary ammonium compound corresponds to the formula I in which, in addition, the sum of p, q and r is between 1 and 3, with the exception of values between 1.40 and 1.85.

2. Base according to Claim 1, characterized in that i is equal to 0.

3. Base according to Claim 2, characterized in that, if m is equal to 1, j is between 1 and 18 and k and d are equal to 0.

4. Base according to Claim 3, characterized in that j is between 1 and 4.

5. Base according to Claim 4, characterized in that, if j is equal to 1, $R_5$ is a hydrocarbon chain having from 7 to 17 carbon atoms.

6. Base according to Claim 2, characterized in that, if m is equal to 2, k and d are equal to 0 and j is between 2 and 12.

7. Base according to Claim 2, characterized in that, if m is equal to 2, k is equal to 1, j is between 4 and 12, d is equal to 0 and $R_5$ is a hydrocarbon chain having 7 or 9 carbon atoms.

8. Base according to Claim 2, characterized in that m is equal to 3, j is equal to 3 or 4, k and d are equal to 0 and $R_5$

is a hydrocarbon chain having 7 or 9 carbon atoms.

9. Base according to Claim 2, characterized in that, if m is equal to 3, j is equal to 6 and k and d are equal to 0.

10. Base according to Claim 2, characterized in that, if m is equal to 4, j is equal to 5 and k and d are equal to 0.

11. Base according to any one of Claims 1 to 10, characterized in that it comprises 60 to 99% by weight of at least one compound of the formula I and 40 to 1% by weight of at least one compound of the formula II.

12. Suppling composition, characterized in that it comprises a surfactant base according to any one of Claims 1 to 11.

**Patentansprüche**

1. Tensidaktives Basismittel, mit zumindest einer quartären kationischen Ammoniumverbindung, die der folgenden Formel I entspricht:

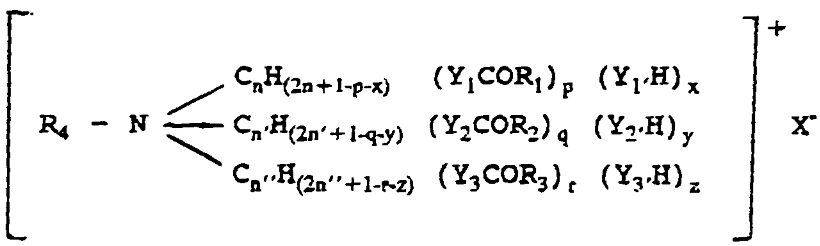

(I)

in der

- n, n', n" ganze Zahlen, jedoch nicht Null bedeuten
- p, q, r, x, y, z ganze Zahlen oder Null bedeuten
- die Summe von n, n' und n" im Bereich von 4 bis 12 liegt
- die Summe von p, q, r, x, y und z im Bereich von 1 bis 4 liegt
- $Y_1$, $Y_{1'}$, $Y_2$, $Y_{2'}$, $Y_3$, $Y_{3'}$ funktionelle Gruppen sind, jede ausgewählt aus den Gruppen Oxy, N und NH
- $R_1$, $R_2$, $R_3$ jeweils eine lange Alkyl- oder Alkylenkohlenwasserstoffkette mit 5 bis 23 Kohlenstoffatomen ist
- $R_4$ eine kurze Alkylkette ist, die 1 bis 5 Kohlenstoffatome aufweist, und
- X ein Gegenion ist, insbesondere ausgewählt aus den Halogeniden, den Karbonaten und den Alkylsulfaten,
- wobei das Basismittel als Lösungsmittel zumindest eine Verbindung enthält, die der folgenden Formel II entspricht:

$$(R_5 - E)_m - C_j H_{2(j+1)-m-2d+i} O_{k+i} \qquad \text{(II)}$$

in der:
- $R_5$ eine lange gesättigte oder ungesättigte Kohlenwasserstoffkette ist, die 5 bis 23 Kohlenstoffatome aufweist
- E eine Funktion

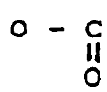
oder
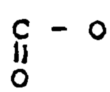

ist
- m eine ganze Zahl im Bereich von 1 bis 8 ist
- j eine ganze Zahl im Bereich von 1 bis 36 ist
- k eine ganze Zahl oder Null ist
- i eine ganze oder Null ist
- d 0, 1 oder 2 ist,

dadurch gekennzeichnet, daß die quartäre kationische Ammoniumverbindung der Formel I entspricht, in der darüber hinaus die Summe von p, q und r im Bereich von 1 bis 3 liegt, mit Ausnahme der Werte von 1,40 bis 1,85.

2. Basismittel nach Anspruch 1, dadurch gekennzeichnet, daß i gleich 0 ist.

3. Basismittel nach Anspruch 2, dadurch gekennzeichnet, daß, falls m gleich 1 ist, j im Bereich von 1 bis 18 liegt und k und d gleich 0 sind.

4. Basismittel nach Anspruch 3, dadurch gekennzeichnet, daß j im Bereich von 1 bis 4 liegt.

5. Basismittel nach Anspruch 4, dadurch gekennzeichnet, daß, falls j gleich 1 ist, $R_5$ eine Kohlenwasserstoffkette mit 7 bis 17 Kohlenstoffatomen ist.

6. Basismittel nach Anspruch 2, dadurch gekennzeichnet, daß, falls m gleich 2 ist, k und d gleich 0 sind, und j im Bereich von 2 bis 12 liegt.

7. Basismittel nach Anspruch 2, dadurch gekennzeichnet, daß, falls m gleich 2 ist, k gleich 1 ist, j im Bereich von 4 bis 12 liegt, d gleich 0 ist, und $R_5$ eine Kohlenwasserstoffkette mit 7 oder 9 Kohlenstoffatomen ist.

8. Basismittel nach Anspruch 2, dadurch gekennzeichnet, daß m gleich 3 ist, j gleich 3 oder 4 ist, k und d gleich 0 sind, und $R_5$ eine Kohlenwasserstoffkette mit 7 oder 9 Kohlenstottatomen ist.

9. Basismittel nach Anspruch 2, dadurch gekennzeichnet, daß, falls m gleich 3 ist, j gleich 6 ist, und k und d gleich 0 sind.

10. Basismittel nach Anspruch 2, dadurch gekennzeichnet, daß, falls m gleich 4 ist, j gleich 5 ist, und k und d gleich 0 sind.

11. Basismittel nach einem der Ansprüche 1 bis 10, dadurch gekennzeichnet, daß es 60 bis 99 Gew.-% zumindest einer Verbindung der Formel I und 40 bis 1 Gew.-% zumindest einer Verbindung der Formel II enthalt.

12. Weichmacherzusammensetzung, dadurch gekennzeichnet, daß diese ein tensidaktives Basismittel nach einem der Ansprüche 1 bis 11 enthält.